(19) Europäisches Patentamt
European Patent Office
Office européen des brevets

(11) Publication number: 0 087 294
B1

(12) **EUROPEAN PATENT SPECIFICATION**

(45) Date of publication of patent specification: **19.04.89**

(51) Int. Cl.$^4$: **A 61 L 2/08,** C 08 K 5/00, C 08 K 5/34

(21) Application number: **83300861.8**

(22) Date of filing: **18.02.83**

(54) **Radiation stabilization of polymeric material.**

(30) Priority: **23.02.82 US 351398**

(43) Date of publication of application:
**31.08.83 Bulletin 83/35**

(45) Publication of the grant of the patent:
**19.04.89 Bulletin 89/16**

(84) Designated Contracting States:
**BE DE FR GB IT LU NL SE**

(56) References cited:
**EP-A-0 007 736**
**US-A-4 274 932**

(73) Proprietor: **Becton, Dickinson and Company**
**Mack Centre Drive P.O. Box 2224**
**Paramus New Jersey 07652-1149 (US)**

(72) Inventor: **Williams, Joel L.**
**1306 Walnut Street**
**Cary North Carolina 27511 (US)**
Inventor: **Dunn, Terry S.**
**1300 Canterbury Road**
**Raleigh North Carolina 27608 (US)**

(74) Representative: **Ruffles, Graham Keith et al**
**MARKS & CLERK 57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 087 294 B1**

**Description**

The present invention relates generally to semi-crystalline polymers which are suitable for stabilization by irradiation sterilization. More particularly, the present invention relates to additives for semi-crystalline polymers, such as propylene polymer compositions, to improve the properties of the polymer when subject to irradiation.

Semi-crystalline polymeric materials, such as polypropylene, are often employed in articles where it is necessary to subject the article to irradiation sterilization. The polymeric material without additives is subject, however, to degradation, embrittlement and discolouration during or subsequent to such irradiation. Many additives have been proposed for such semi-crystalline polymeric materials to inhibit the discolouration and degradation resulting from the irradiation sterilization treatment. US Patent 4,100,185, for example, discloses irradiation sterilization of semi-crystalline polymers wherein the semi-crystalline polymer has incorporated therein a non-crystalline mobilizing additive which increases the free volume of the polymer thereby to prevent embrittlement of the polymer during and subsequent to the irradiation.

United States Patent 4,274,932 is directed to a process for sterilizing a semi-crystalline polymer which process includes the steps of subjecting a semi-crystalline polymer to a sterilizing amount of high energy irradiation. The semi-crystalline polymer has a specific crystalline content of from 20 to 90% and the ratio of the weight average molecular weight to the number average molecular weight is no greater than 9. The polymer, furthermore, has incorporated therein a mobilizing amount of a non-crystalline liquid mobilizing additive which increases the free volume of the polymer and retains the flexibility thereof.

United States Patent 3,940,325 is directed to radiation sterilized shaped articles of olefin polymers. The polymers contain from 0.01 to 0.5 per cent by weight based on the weight of the olefin polymer of octadecyl 3,5-di-t-butyl-r-hydroxyhydrocinnamate and/or tetrakis methane.

United States Patent 3,537,967 is directed to a radiation sterilized article having improved colour which is manufactured from a polypropylene polymer with a substantial crystalline content which has added to it up to one per cent of an ester of thiodipropionic acid as a stabilizer.

European Patent Application 79301347.5, Specification 7736, is directed to polyolefin articles, especially polypropylene hypodermic syringes, which are made more resistant to the yellow discolouration and embrittlement which accompanies sterilization by gamma irradiation. Resistance is imparted by the addition to the polyolefin of a heterocyclic hindered amine.

While the prior art, as outlined above, disclosed many efforts to provide an additive to stabilize semi-crystalline polymers to prevent discolouration and degradation when subjected to irradiation sterilization, the problem persists for semi-crystalline polymers used in medical articles. The present invention is directed to an improvement in the irradiation sterilization of a semi-crystalline polymer by incorporating with the polymer a combination of stabilizing agent comprising a hindered amine and a mobilizing additive.

In accordance with one aspect of the present invention there is provided a semi-crystalline polymer which has incorporated therein a combination of stabilizing agents comprising a heterocyclic hindered amine and a non-crystalline mobilizing additive. It has been found that the combination of a mobilizing additive and a heterocyclic hindered amine incorporated into a semi-crystalline polymer provides a synergistic improvement in the irradiation stability of such polymers. Accordingly, such semi-crystalline polymers having a combination stabilizer including a non-crystalline mobilizing additive and a heterocyclic hindered amine when subjected to sterilizing irradiation has increased stability to embrittlement and to discolouration during and subsequent to such irradiation. The combination stabilizer is employed in the absence of an antagonistic sulphur compound which is a long chain mercaptan or sulphide, or a dialkyl thiodialkanoate. The non-crystalline mobilizing additive is a low molecular weight substance which is miscible with the polymeric material and is also compatible therewith. The mobilizing additive is a substance which increases the free volume of the polymer, and therefore, also lowers the density of the polymer.

The mobilizing additive can be any one of a wide variety of liquids which increase the total free volume of the polymer. The term "liquid", as used herein, includes highly viscous substances, commonly referred to as greases. In general, such mobilizing additives have a density of from 0.6 to 1.9 g/cm$^3$ and preferably from 0.6 to 1.1 g/cm$^3$. The mobilizing additive has a low molecular weight, with the average molecular weight being on the order of from about 100 to about 10,000 grams/mole and preferably from about 100 to about 5,000 grams/mole.

Representative examples of suitable mobilizing additives include hydrocarbon oils, halogenated hydrocarbon oils, phthalicester oils, vegetable oils, silicon oils, low molecular weight non-crystalline polymer greases, such as hydrocarbon polymer greases, low molecular weight polyester greases, polyarylether greases, etc. The preferred mobilizer is a liquid mobilizer which is not highly viscous, in particular a hydrocarbon oil or phthalicester oil.

The mobilizing additive is incorporated into the polymer in a mobilizing amount. The mobilizing additive is generally present in an amount of from about 0.01 per cent to about 50 per cent by weight of the polymer and preferably from about 0.1 per cent to about 20 per cent. All percentages used herein are by weight and are based on the weight of semi-crystalline polymers unless expressly designated otherwise.

The hindered amines preferably comprise a 5- or 6-membered heterocyclic ring containing the

hindered amine nitrogen and optionally another hetero atom preferably nitrogen or oxygen. If the hindered amine is a tertiary amine, the tertiary group may be, for example, an alkyl, aralkyl, alkaryl or alicyclic group containing 1 to 12 carbon atoms. One or more of the substituents of the hindered amine may itself be a hindered amine so that the tertiary group may be used to link a plurality of hindered amines. The hindering groups are preferably alkyl groups containing 1 to 4 carbon atoms and most preferably all four groups are methyl. The most preferred hindered amines comprise 2,2,4,4-tetramethyl piperidine derivatives.

The hindered amine is preferably bonded to a carrier moiety which should have little if any inhibiting effect on the chemical activity of the hindered amine. Reasonably inert carriers include aromatic compounds (for example those based on the benzene, imidazole or triazine rings), saturated hydrocarbon compounds, esters or amides of carboxylic acids, ketones and ether, thioether, sulphinyl or sulphone groups. Preferably, the carriers can be used to link together a plurality of hindered amines and hence the tertiary groups may be regarded as carriers too.

Examples of hindered amines linked by diesters or ketones include:

(a) di-(2,2,6,6-tetramethyl-4-piperidyl) sebacate (see formula I) where it is the sebacate which is the carrier;

(b) di-(2,2,6,6-tetramethyl-4-piperidyl)-1-(3,5-ditertiarybutyl-4-hydroxyphenylmethyl)-1,1-pentanedicarboxylate (see formula II) where it is the pentanedicarboxylate which is the carrier;

(c) the condensate of succinic acid and N-(2-hydroxypropyl)-2,2,6,6-tetramethyl-4-hydroxypiperidine (see formula III) where both the succinate moiety and the tertiary propoxy groups combine to form carriers (the condensate preferably contains 6 to 20 hindered amine groups), and

(d) 1,4-di-(2,2,6,6-tetramethyl-4-piperidyl)-2,3-butanedione (see formula IV) where it is the butanedione which is the carrier.

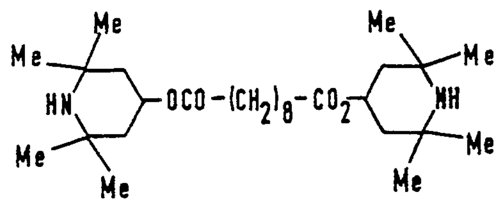
(I)

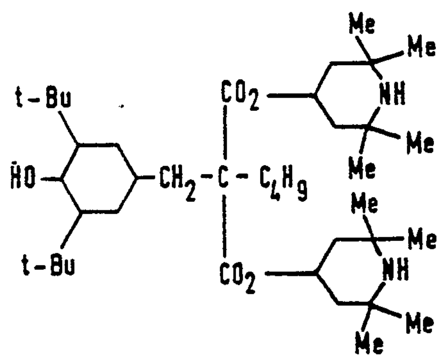
(II)

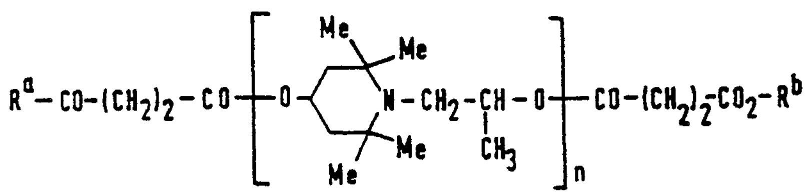
(III)

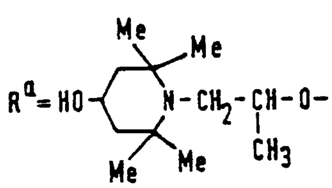

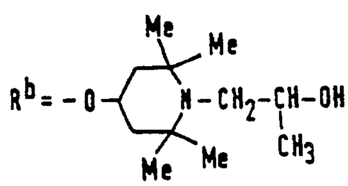

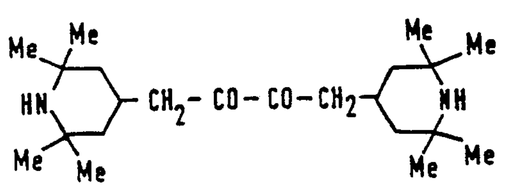
(IV)

The semi-crystalline polymer can be, for example, a homopolymer of ethylene (low or high density polyethylene), propylene, butene-1 or 4-methylpentene-1 or a copolymer of two or more of these monomers. Preferred copolymers are copolymers of propylene and from 7 to 20 per cent (by weight of the copolymer) of ethylene, when made by injecting ethylene into the latter stages of an otherwise homopolymerization of propylene, or from 0.5 to 10 per cent of ethylene when made by random copolymerization. Copolymers of ethylene with up to 30 per cent of vinyl acetate, methyl, ethyl or butyl (including tertiary butyl) acrylate or methacrylates or acrylic or methacrylic acids may also be used. The preferred semi-crystalline polymer is polypropylene having specific crystalline content of from 20 to 90 per cent.

The composition may contain stabilizing amounts (eg, 0.01 to 2 per cent by weight of the polymer) of light stabilizers (eg benzotriazoles) and/or phenolic antioxidants of the kind used in polyolefins, for example, n-octadecyl 3-(3,5-ditertiarybutyl-4-hydroxyphenyl) propionate. However, phenolic antioxidants may aggravate discolouration so their use may entail sacrifice of some of the improvement in discolouration. The compositions may also contain stabilizing amounts of sulphur compounds of the type known to synergise with phenolic antioxidants in polyolefins, for example, long chain (eg $C_{10}$ to $C_{22}$) mercaptans and sulphides but the preferred compounds are dialkyl thiodialkanoates especially when the alkyl groups contain from 2 to 6 carbon atoms. The use of some of such sulphur compounds reduces the synergistic effect of the combination of stabilizing agents of the present invention and these compounds are therefore excluded. The compositions preferably contain from 0.01 to 10 per cent (usually 0.1 to 0.5 per cent) by weight of organic sulphur compound. Other conventional additives such as pigments or moulding aids may be used.

The heterocyclic hindered amine is added to the semi-crystalline polymer at a level of from about 0.005 to about 0.5 per cent by weight of the polymer, preferably from about 0.5 per cent to about 0.3 per cent. It has furthermore been determined that the weight ratio of the non-crystalline mobilizing additives to the heterocyclic hindered amine should be from about 0.5 to about 20, preferably from about 2 to about 10.

While not wishing to be bound by any theory, it is believed that the mobilizing additive functions to mobilize the amorphous portion of the polymer while at the same time the presence of the hindered amine captures radicals in the accessible portions of the polymer. The net effect is believed to be that the mobilizing additive increases the radical termination reaction and prevents these reactions from affecting the bulk portion of the polymer to prevent or minimize degradation and discolouration subsequent to the irradiation. Also, the mobilizing additive aids the hindered amine in capturing radicals formed during the irradiation of the polymer.

Further feature of the present invention are illustrated by the following Example. Reference is made to the accompanying drawings, in which:

Figure 1 is a plot of irradiation breakdown with time at various levels of irradiation for a polymer having only a hindered amine present as a stabilizer.

Figure 2 is a plot of irradiation breakdown with time at various levels of irradiation for a polymer having only a mobilizing additive present as a stabilizer.

Figure 3 is a plot of irradiation breakdown with time at various levels of irradiation for a polymer having a combination of a hindered amine and a mobilizing additive present as a stabilizer.

Figure 4 is an irradiation breakdown at various levels of irradiation for a polymer containing the combination of stabilizing agents of the invention and also containing a commonly used sulphur compound, dilauryl-thiodipropionate (DLTDP).

## Example

Polypropylene containing 0.1% hindered amine (formula I) and having a melt flow of 12 and a ratio of weight to number average molecular weight ($m_w/m_n$) of 2.8 was irradiated with 2, 3, 5, and 7 megarads of gamma radiation ($Co^{60}$). Immediately following irradiation, the sample was brittle and continued to embrittle with age as shown in Figure 1.

In a separate experiment, 4.7% of a mobilizing additive (hydrocarbon oil manufactured by Witco Company and identified by the trade name WITCO 300) was added to the polypropylene without the addition of hindered amine. In this case, an improvement in irradiation stability over the polypropylene with hindered amine alone was observed as shown in Figure 2 where embrittlement was measured after 5 megarads.

A dramatic improvement in irradiation stability was observed, however, when the 0.1% hindered amine (formula I) and 4.7% mobilizing additive (WITCO 300 hydrocarbon oil) were added to the polypropylene as shown in Figure 3. In this case, the stabilized polypropylene could withstand 7 megarads without embrittlement even after 9 months of aging following irradiation. In addition, the samples containing mobilizing additive and hindered amine did not discolour with irradiation.

Thus, the combination of hindered amine with mobilizing additive produced an outstanding irradiation stabilizer package for polypropylene as disclosed herein.

The addition of certain thioesters (especially dilaurylthiodipropionate) to the polypropylene containing hindered amine (0.1% formula I) and mobilizing additive (4.7% WITCO 300 hydrocarbon oil) produced a negative effect as shown in Figure 4.

## Claims

1. An irradiation sterilizable composition stabilized against discolouration and degradation comprising a semi-crystalline polymer having incorporated therein a synergistic stabilizing mixture of a mobilizing amount of a non-crystalline liquid mobilizing additive which increases the free volume of the polymer and also having incorporated therein a sterically hindered amine or its N-oxide, N-hydroxide or N-nitroxide salt, with the amine nitrogen being contained in a carbon-nitrogen-carbon chain which forms part of a nonaromatic heterocyclic ring and wherein each of the two carbon atoms of the said chain is bonded either to two alkyl groups which are the same or different and each have 1 to 12 carbon atoms or to an alicyclic group containing 4 to 9 carbon atoms, in the absence of an antagonistic sulphur compound which is a long chain mercaptan or sulphide, or a dialkyl thiodialkanoate.

2. A composition in accordance with claim 1, wherein the semi-crystalline polymer is a homopolymer of ethylene, propylene, butene-1, or 4-methylpentene-7, or a copolymer of two or more of these monomers.

3. A composition in accordance with claim 1 or 2, wherein the mobilizing additive has a density of from 0.6 to 1.9 g/cm$^3$ and a molecular weight of from 100 to 10,000.

4. A composition in accordance with any preceding claim wherein the mobilizing additive is selected from hydrocarbon oils, vegetable oils, silicon oils, low molecular weight non-crystalline polymer greases, such as hydrocarbon polymer greases, low molecular weight polyester greases and polyarylether greases.

5. A composition in accordance with claim 4 wherein the mobilizing additive is a hydrocarbon oil.

6. A composition in accordance with any preceding claim wherein the mobilizing additive is present in an amount from 0.01% to 50% by weight of the composition.

7. A composition in accordance with any preceding claim wherein the hindered amine is present at a level of from 0.005 to 0.5% by weight of the composition.

8. A composition in accordance with any preceding claim wherein the weight ratio of the mobilizing additive to the heterocyclic amine is from 0.5:1 to 20:1.

9. A composition in accordance with any preceding claim, which does not contain any dilaurylthiopropionate.

10. An article prepared from a composition according to any preceding claim, after the article has been subjected to irradiation sterilization.

## Patentansprüche

1. Gegen Verfärbung und abbaustabilisierte strahlungssterilisierbare Masse mit einem halbkristallisierbarem Polymer, in welches eine synergistische Stabilisiermischung aus einer mobilisierenden Menge eines nicht kristallinen flüssigen mobilisierenden Zusatzes, das das freie Volumen des Polymers erhöht, und auch ein sterisch gehindertes Amin oder sein N-Oxyd, N-Hydroxyd oder N-Nitroxydsalz, das den Aminstickstoff in einer Kohlenstoff-Stickstoff-Kohlenstoff-Kette, welche Teil eines nicht-aromatischen heterocyclischen Ringes bildet und worin jedes der zwei Kohlenstoffatome der genannten Kette entweder an zwei gleiche oder verschiedene Alkylgruppen mit je 1 bis 12 Kohlenstoffatomen oder an eine alicyclische Gruppe mit 4 bis 9 Kohlenstoffatomen gebunden ist, enthält, eingearbeitet ist, in Abwesenheit einer antagonistischen Schwefelverbindung, welche ein langkettiges Mercaptan oder Sulfid oder ein Dialkylthioalkanoat ist.

2. Masse nach Anspruch 1, worin das halbkristalline Polymer ein Homopolymer von Äthylen, Propylen, Buten-1 oder 4-Methylpenten-7 oder Copolymer von zwei oder mehr dieser Monomere ist.

3. Masse nach Anspruch 1 oder 2, worin der mobilisierende Zusatz eine Dichte von 0,6 bis 1,9 g/cm$^3$ und ein Molekulargewicht von 100 bis 10 000 hat.

4. Masse nach irgendeinem der vorhergehenden Ansprüche, worin der mobilisierende Zusatz aus Kohlenwasserstoffölen, Pflanzenölen, Siliconölen, niedermolekularen nichtkristallinen Polymerfetten, wie Kohlenwasserstoffpolyfetten, niedermolekularen Polyesterfetten und Polyarylätherfetten ausgewählt ist.

5. Masse nach Anspruch 4, worin der mobilisierende Zusatz ein Kohlenwasserstofföl ist.

6. Masse nach einem der vorhergehenden Ansprüche, worin der mobilisierende Zusatz in einer Menge von 0,01 Gew.-% bis 50 Gew.-% der Masse zugegen ist.

7. Masse nach einem der vorhergehenden Ansprüche, worin das gehinderte Amin in einer Menge von 0,05 bis 0,5 Gew.-% der Masse zugegen ist.

8. Masse nach einem der vorhergehenden Ansprüche, worin das Gewichtsverhältnis des mobilisierenden Zusatzes zum heterocyclischen Amin 0,5:1 bis 20:1 ist.

9. Masse nach einem der vorhergehenden Ansprüche, welche kein Dilaurylthiopropionat enthält.

10. Gegenstand, hergestellt aus einer Masse nach einem der vorhergehenden Ansprüche, nachdem der Gegenstand einer Strahlungssterilisation unterworfen worden ist.

## Revendications

1. Composition stérilisable par irradiation, stabilisée vis-à-vis d'un changement de couleur et d'une dégradation, comprenant un polymère semi-cristallin dans lequel a été incorporé un mélange stabilisant

synergique d'une quantité mobilisante d'un additif liquide non-cristallin de mobilisation, qui augmente le volume libre du polymère, et dans lequel est incorporée une amine à empêchement stérique ou son sel N-oxyde, N-hydroxyde ou N-nitroxyde, l'azote d'amine étant contenu dans une chaîne carbone-azote-carbone faisant partie d'un noyau hétérocyclique non-aromatique, et où chacun des deux atomes de carbone de ladite chaîne est fixé soit à deux groupes alkyle qui sont identiques ou différents, et dont chacun a de 1 à 12 atomes de carbone, soit à un groupe alicyclique contenant de 4 à 9 atomes de carbone, en l'absence d'un composé sulfuré antagoniste qui soit un mercaptan ou un sulfure à longue chaîne ou un thiodialcanoate de dialkyle.

2. Composition selon la revendication 1, dans laquelle le polymère semi-cristallin est un homopolymère d'éthylène, de propylène, de butène-1 ou de 4-méthylpentène-7, ou un copolymère d'au moins deux de ces monomères.

3. Composition selon la revendication 1 ou 2, dans laquelle l'additif de mobilisation a une masse volumique de 0,6 à 1,9 g/cm$^3$ et une masse moléculaire de 100 à 10 000.

4. Composition selon l'une quelconque des revendications précédentes, dans laquelle l'additif de mobilisation est choisi parmi les huiles hydrocarbonées, les huiles végétales, les huiles de silicone, les graisses polymères non-cristallines à faible masse moléculaire, comme les graisses polymères hydrocarbonées, les graisses à base de polyesters à faible masse moléculaire et les graisses à base de polyaryléther.

5. Composition selon la revendication 4, dans laquelle l'additif de mobilisation est une huile hydrocarbonée.

6. Composition selon l'une quelconque des revendications précédentes, dans laquelle l'additif de mobilisation est présent en une quantité de 0,01 à 50% en poids par rapport à la composition.

7. Composition selon l'une quelconque des revendications précédentes, dans laquelle l'amine à empêchement stérique est présente en une quantité de 0,005 à 0,5% en poids par rapport à la composition.

8. Composition selon l'une quelconque des revendications précédentes, dans laquelle le rapport pondéral de l'additif de mobilisation à l'amine hétérocyclique est compris entre 0,5:1 et 20:1.

9. Composition selon l'une quelconque des revendications précédentes, ne contenant pas de thiopropionate de dilauryle.

10. Article préparé à partir d'une composition selon l'une quelconque des revendications précédentes, après que l'article a été soumis à une stérilisation par irradiation.

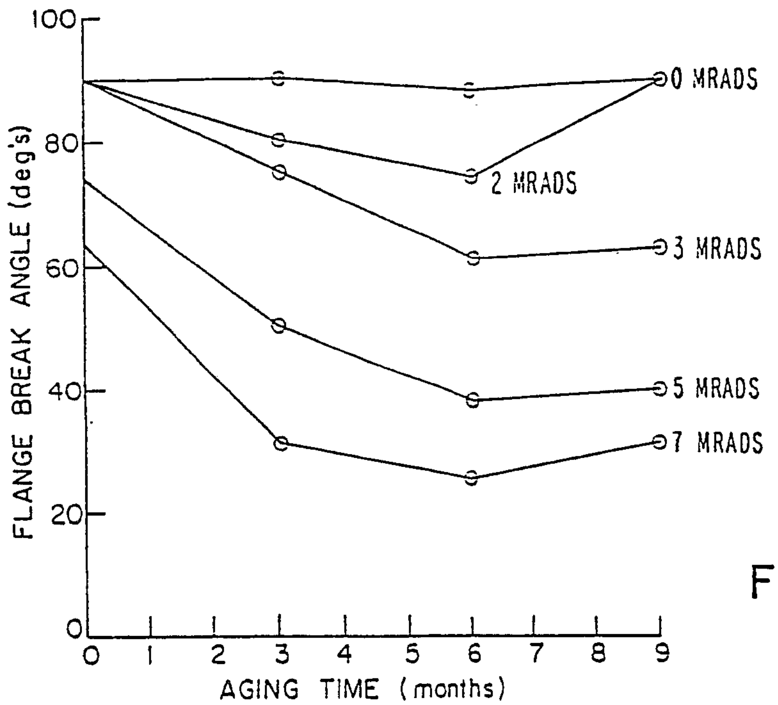
100
80
60
40
20
0
FLANGE BREAK ANGLE (deg's)
0 MRADS
2 MRADS
3 MRADS
5 MRADS
7 MRADS
0 1 2 3 4 5 6 7 8 9
AGING TIME (months)

FIG. 1

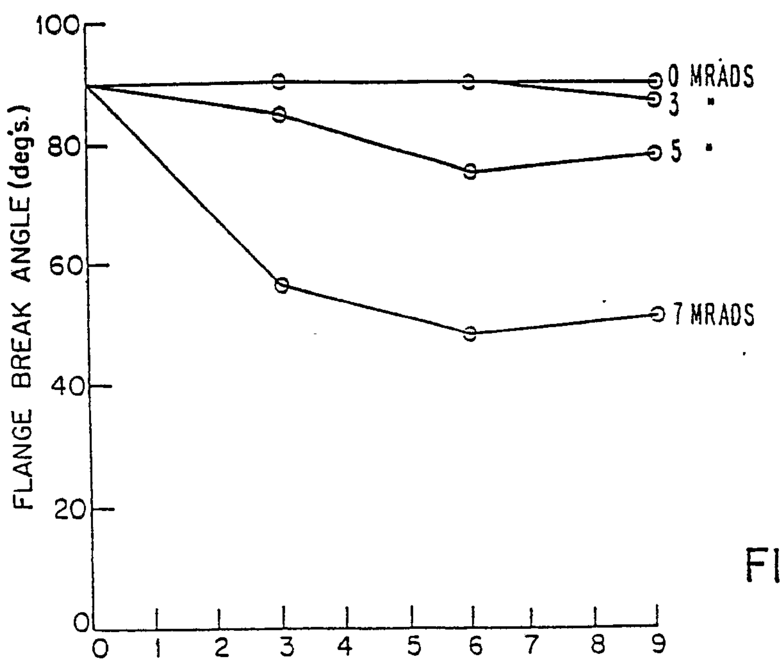
100
80
60
40
20
0
FLANGE BREAK ANGLE (deg's.)
0 MRADS
3 "
5 "
7 MRADS
0 1 2 3 4 5 6 7 8 9

FIG. 2

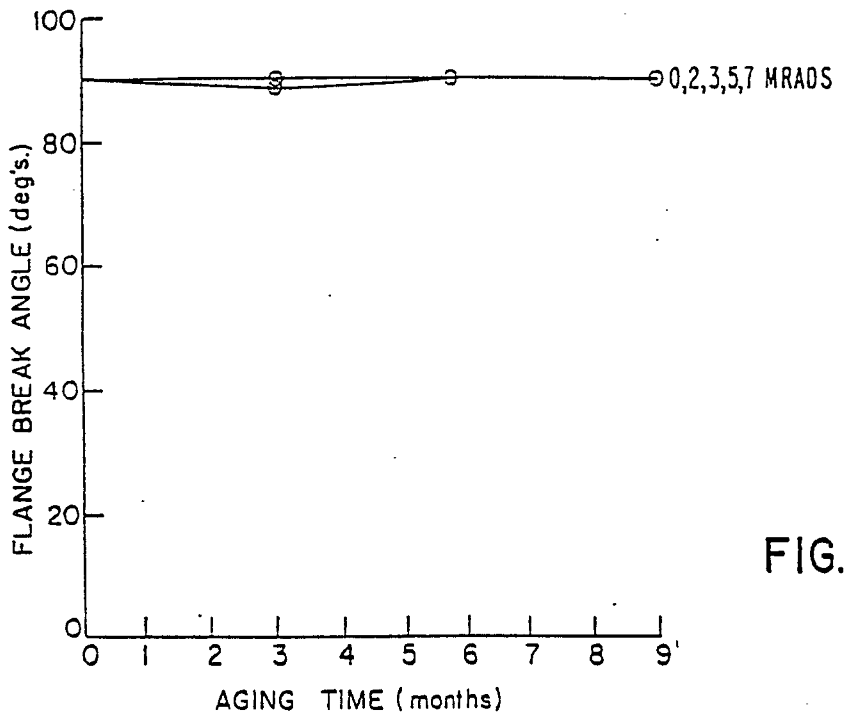

FLANGE BREAK ANGLE (deg's.)
100
80
60
40
20
0
0,2,3,5,7 MRADS
0 1 2 3 4 5 6 7 8 9
AGING TIME (months)


FIG. 3

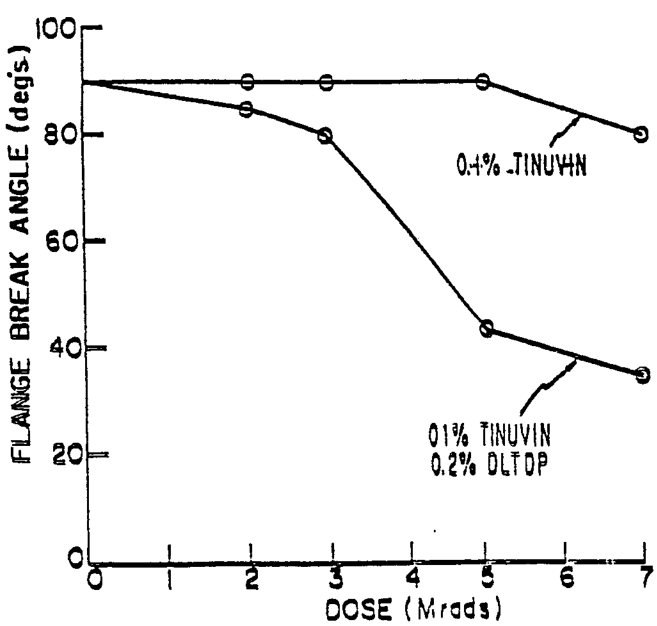

FLANGE BREAK ANGLE (deg's.)
100
80
60
40
20
0
0.4% TINUVIN
01% TINUVIN
0.2% DLTDP
0 1 2 3 4 5 6 7
DOSE (Mrads)


FIG. 4